# EUROPEAN PATENT SPECIFICATION

(11) **EP 3 571 851 B1**
(45) Date of publication and mention of the grant of the patent: **09.12.2026**
(21) Application number: 18741229.1
(22) Date of filing: 05.01.2018
(51) Int. Cl.: H04R 1/10, H04R 25/00, H04R 25/02, A61F 11/08

(54) **EARPIECE INTRA-AURICULAR SUPPORT SYSTEM**
INTRA-AURIKULÄRES STÜTZSYSTEM FÜR EIN OHRSTÜCK
SYSTÈME DE SUPPORT INTRA-AURICULAIRE D'ÉCOUTEUR

(30) Priority: 20.01.2017 US 201715411901
(43) Date of publication of application: 27.11.2019
(73) Proprietor: Decibullz LLC, Fort Collins, CO 80525 (US)
(72) Inventor: KIRKPATRICK, Kyle, J., Loveland CO 805838 (US)
(74) Representative: Kador & Partner Part mbB
(86) International application number: PCT/US2018/012653
(87) International publication number: WO 2018/136247

(56) References cited:
- CN-B- 102 065 806
- US-A- 4 870 688
- US-A1- 2009 028 356
- US-A1- 2009 214 072
- US-A1- 2010 086 158
- US-A1- 2012 057 739
- US-A1- 2012 243 701
- US-A1- 2014 119 589
- US-A1- 2015 146 909
- US-A1- 2016 373 852
- US-A1- 2017 311 069
- ANONYMOUS: "Wacker Silicone Elastomers: Purity and Future Proof", WACKER, March 2014 (2014-03-01), pages 1 - 35, XP055519670, Retrieved from the Internet <URL:http://www.ringierevents.cn/eve_files/files/4db83d3c99c44b18b6b9d38f23a0ac98.pdf> [retrieved on 20180203]

## Description

This International Patent Cooperation Treaty Patent Application is a continuation of United States Non-Provisional Patent Application No. 15/411,901, filed January 20, 2017.

### I. TECHNICAL FIELD

An earpiece including an intra-auricular support positionable in an auricle of an ear, a projection element extending a distance outward of the intra-auricular support, and a flexible body coupled about the projection element and configured to engage an external ear canal of an ear concurrent with the intra-auricular support positioned in the auricle of the ear, and a moldable earpiece material moldable about the intra-auricular support to conform to the auricle of an ear and curable to provide a fixed configuration of the earpiece.

### II. BACKGROUND

Because conventional earpieces may not be configured to the auricle of the ear of a wearer, the earpiece may not stay in fixed engagement with the auricle of the ear, or the earpiece may not correctly align with the external ear canal, or the earpiece may be uncomfortable for the wearer to position into or retain in the auricle of the ear.

Additionally, a wide variety of earplugs or in-ear devices to protect against or deliver sound to the ear such as medical equipment, headsets, hearing aids, cellular telephones, and the like, include, in part, in-ear devices such as earphones, earplugs, earbuds, ear tips, ear tubes, or the like, not configured to the auricle of an individual wearer's ear. US 2015/146909 describes a moldable earpiece that facilitates engagement in the outer ear. US 2012/243701 describes an earplug which conforms to the ear concha and extends into the ear canal. US 2014/119589 describes an earbud with an injection-molded attachment in a shark fin design. US 2012/057739 describes an earpiece configured to fit within the concha. CN 102 065 806 describes a hearing protection earpiece with a moldable element for a portion of the ear canal and a pressure pad.

The instant invention provides the advantages of a moldable earpiece conformed to the auricle of the wearer's ear, and which can retain or removably retain an in-ear device to overcome in whole or in part certain of the forgoing disadvantages associated with conventional in-ear devices.

### III. DISCLOSURE OF THE INVENTION

Accordingly, a broad object of the invention can be to provide an earpiece as defined in the independent claims, including an intra-auricular support engagable to an auricle of an ear, a projection element outwardly extending from the inner surface of the intra-auricular support, a flexible body disposed about the projection element and disposable inside the external ear canal of an ear concurrent with the intra-auricular support engaged with the auricle of the ear, and a moldable earpiece material moldable about the intra-auricular support to conform to the auricle of the ear and curable to provide a fixed configuration of the earpiece.

Naturally, further objects of the invention are disclosed throughout other areas of the specification, drawings, photographs, and claims.

### IV. BRIEF DESCRIPTION OF THE DRAWINGS

FIGURE 1 is a perspective view of a particular embodiment of an intra-auricular support, a projection element coupled to the intra-auricular support, and a flexible body coupled about the projection element.
FIGURE 2 is a side view of the particular embodiment of the intra-auricular support, the projection element, and the flexible body.
FIGURE 3 is a front view of the particular embodiment of the intra-auricular support, the projection element, and the flexible body.
FIGURE 4 is an exploded perspective view of the particular embodiment of an intra-auricular support, projection element, and flexible body.
FIGURE 5 is a cross sectional side view 5-5 of the particular embodiment of an intra-auricular support, projection element, and flexible body shown in Figure 3.
FIGURE 6A is a particular embodiment of a one-part moldable earpiece material "A".
FIGURE 6B is another particular embodiment of a moldable earpiece material including moldable agent "A" and a curing agent "B" combinable to generate moldable earpiece material "AB".
FIGURE 7 is an illustration of the particular embodiment of an intra-auricular support, projection element, and flexible body having the intra-auricular support engaged with the auricle of the ear concurrent with the flexible body disposed in the external ear canal of an ear.
FIGURE 8 is a particular embodiment of the intra-auricular support having patterned surface elements as an adhesion element.
FIGURE 9A is a particular embodiment of a moldable earpiece material including a tackifier "C" as an adhesion element.
FIGURE 9B is another particular embodiment of a moldable earpiece material having a tackifier "C" as an adhesion element.
FIGURE 10 is a cross sectional side view of an ear and the particular embodiment of an intra-auricular support, projection element, and flexible body which shows the intra-auricular support engaged with the auricle of the ear concurrent with the flexible body disposed in the external ear canal of an ear.
FIGURE 11 is a cross sectional side view of the ear and the particular embodiment of an intra-auricular support, projection element, and flexible body engaged with the ear as shown in Figure 10 and further illustrating a moldable earpiece material A or AB moldably engaged with the intra-auricular support and the auricle of the ear.
FIGURE 12 is a cross sectional side view of the ear and the particular embodiment of an intra-auricular support, projection element, and flexible body engaged with the ear as shown in Figure 11 and further illustrating a moldable earpiece material A or AB molded about the intra-auricular support and to the contour of the auricle of the ear to provide a fixed configuration of the earpiece.
FIGURE 13 is a perspective view of a particular embodiment of a fixed configuration of an earpiece in an ear.
FIGURE 14 is an exploded view of a particular embodiment of an intra-auricular support, a projection element outwardly extending from the intra-auricular support and a flexible body coupled about the projection element and an intra-auricular support conduit disposed in the intra-auricular support configured to removably retain an in-ear device.
FIGURE 15 is a perspective view of the particular embodiment shown in Figure 14 including the intra-auricular support, the projection element, the flexible body, and the intra-auricular support conduit removably retaining an in-ear device.
FIGURE 16 is a cross sectional side view 16-16 of the particular embodiment of the intra-auricular support having the intra-auricular support conduit removably retaining an in-ear device, the projection element, the flexible body.
FIGURE 17 is a cross sectional side view of the ear and the particular embodiment of an intra-auricular support having the intra-auricular support conduit removably retaining an in-ear device, projection element, and the flexible body coupled about the projection element and engaged with the external ear canal and further illustrating a moldable earpiece material A or AB molded about the intra-auricular support and to the contour of the auricle of the ear to provide a fixed configuration of the earpiece.
FIGURE 18 is a perspective view of the particular embodiment of a fixed configuration of the earpiece retaining an in-ear device in an ear.
FIGURE 19 is an exploded view of the particular embodiment of an intra-auricular support having an intra-auricular support conduit configured to removably retain an in-ear device having a body and a sound delivery element with the sound delivery element extendable through the intra-auricular support conduit to provide a projection element, and a flexible body which removably couples to the projection element.
FIGURE 20 is a perspective view of the particular embodiment of an intra-auricular support having an intra-auricular support conduit removably retaining an in-ear device having a body and a sound delivery element with the sound delivery element extending outwardly from the intra-auricular support to provide a projection element, and a flexible body which removably couples to the projection element.
FIGURE 21 is a cross sectional side view 21-21 of the particular embodiment of an intra-auricular support having the intra-auricular support conduit retaining an in-ear device with the sound delivery element extending outwardly from the intra-auricular support to provide the projection element, and the flexible body removably coupled to the projection element.
FIGURE 22 is a cross sectional side view of the ear and the particular embodiment of an intra-auricular support having the intra-auricular support conduit retaining an in-ear device with the sound delivery element extending outwardly from the intra-auricular support to provide the projection element, and the flexible body engaged with the ear and further illustrating a moldable earpiece material A or AB molded about the intra-auricular support and to the contour of the auricle of the ear to provide a fixed configuration of the earpiece.
FIGURE 23 is a perspective view of the particular embodiment of the fixed configuration of the earpiece in an ear.
FIGURE 24 is an exploded view of a particular embodiment of an intra-auricular support having an intra-auricular support conduit which extends a distance outwardly from the outer surface of the intra-auricular support and configured to removably retain an in-ear device having a body and a sound delivery element with the sound delivery element extendable outwardly from the inner surface of the intra-auricular support to provide a projection element, and a flexible body which removably couples to the projection element.
FIGURE 25 is a perspective view of a particular embodiment of an intra-auricular support having an intra-auricular support conduit which extends a distance outwardly from the outer surface of the intra-auricular support and removably retaining an in-ear device having a body and a sound delivery element extending outwardly from the inner surface of the intra-auricular support to provide a projection element, and a flexible body which removably coupled to the projection element.
FIGURE 26 is a cross sectional side view 26-26 of the particular embodiment of an intra-auricular support having the intra-auricular support conduit retaining an in-ear device with the sound delivery element extending outwardly from the intra-auricular support to provide the projection element, and the flexible body removably coupled to the projection element.
FIGURE 27 is a cross sectional side view of the particular embodiment of the intra-auricular support having the intra-auricular support conduit retaining an in-ear device with the sound delivery element extending outwardly from the intra-auricular support to provide the projection element, and the flexible body engaged with the external ear canal of the ear and further illustrating a moldable earpiece material A or AB molded about the intra-auricular support and to the contour of the auricle of the ear to provide a fixed configuration of the earpiece.
FIGURE 28 is a perspective view of the particular embodiment of the fixed configuration of the earpiece in an ear.
FIGURE 29 is an exploded view of the particular embodiment of an intra-auricular support having an intra-auricular support conduit including a flexible elastomer insert configured to removably retain an in-ear device a projection element extending outward from the inner surface of the intra-auricular support, and a flexible body which removably couples to the projection element.
FIGURE 30 is a perspective view of the particular embodiment of an intra-auricular support having an intra-auricular support conduit including a flexible elastomer insert removably retaining an in-ear device, a projection element extending outwardly from the intra-auricular support, and a flexible body removably coupled to the projection element.
FIGURE 31 is a cross sectional side view 31-31 of the particular embodiment of an intra-auricular support shown in Figure 30 having the intra-auricular support conduit including a flexible elastomer insert retaining an in-ear device.

### V. MODE(S) FOR CARRYING OUT THE INVENTION

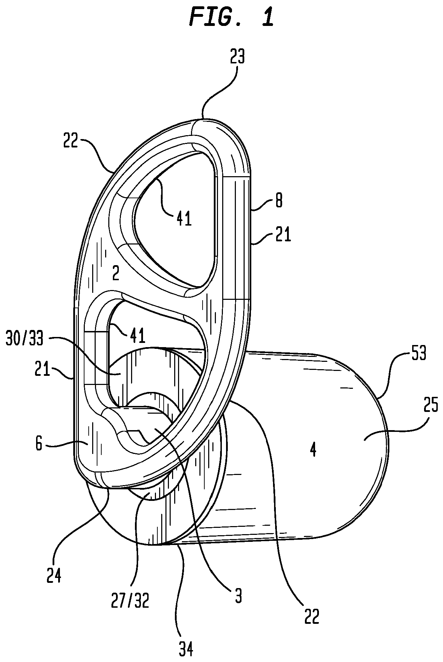

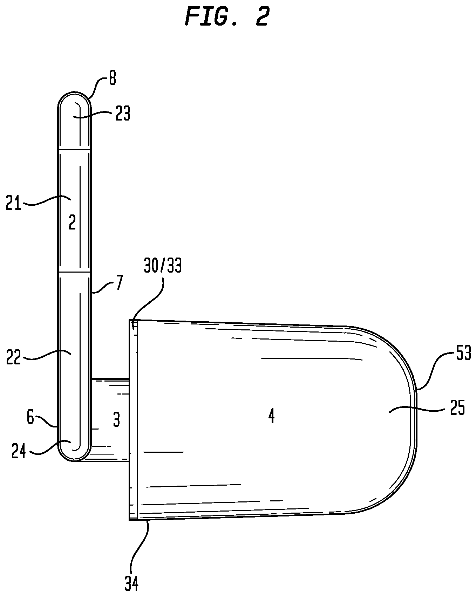

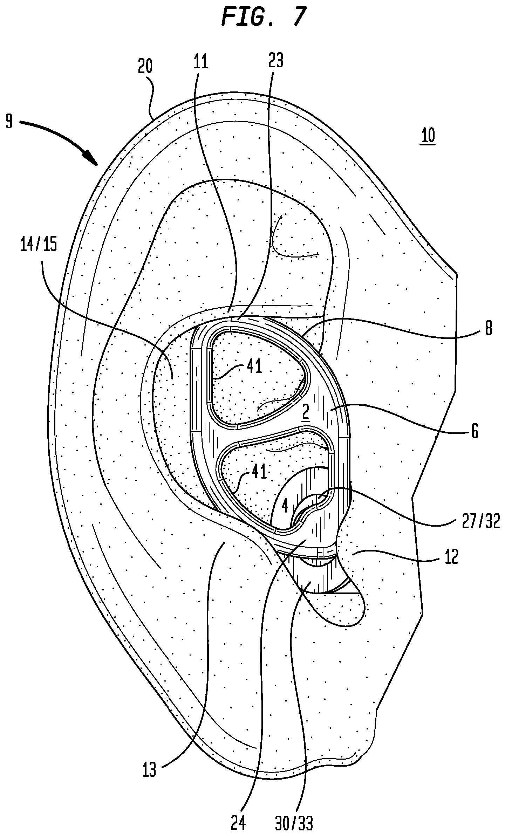

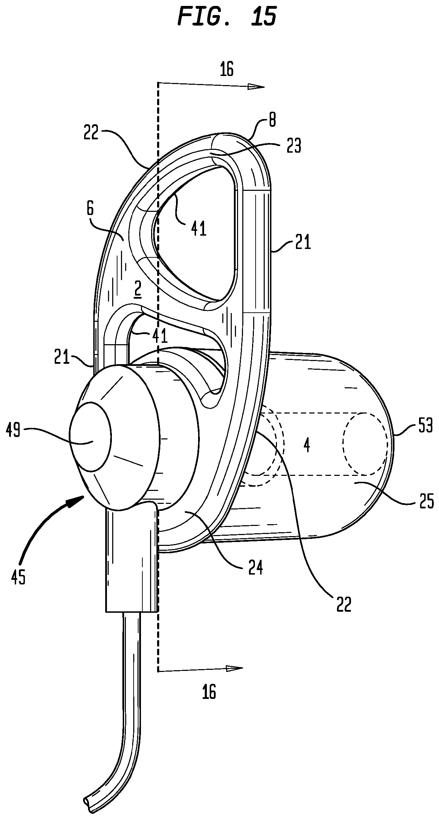

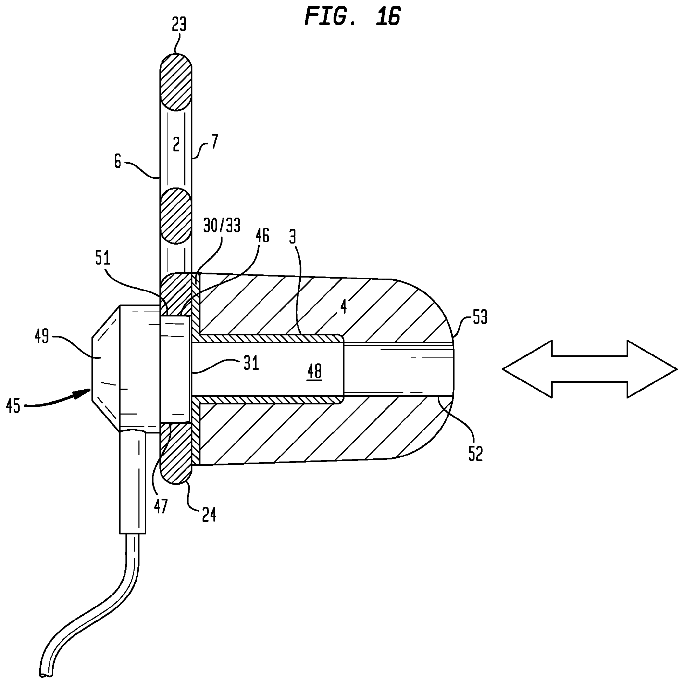

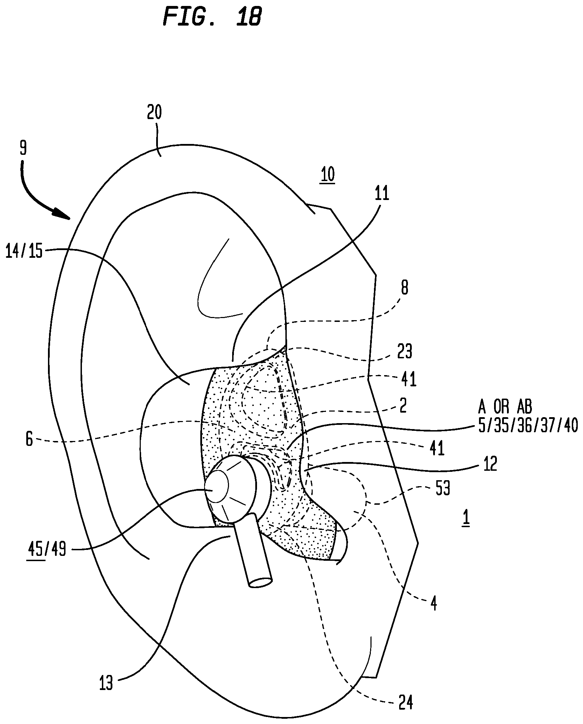

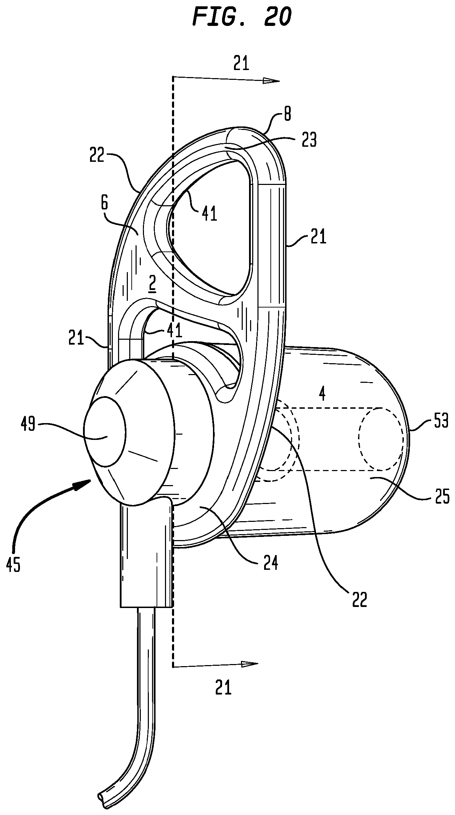

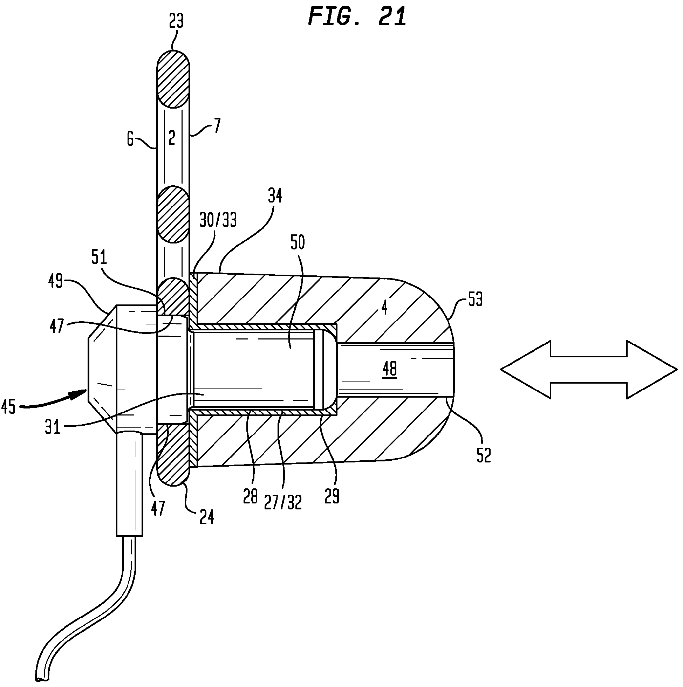

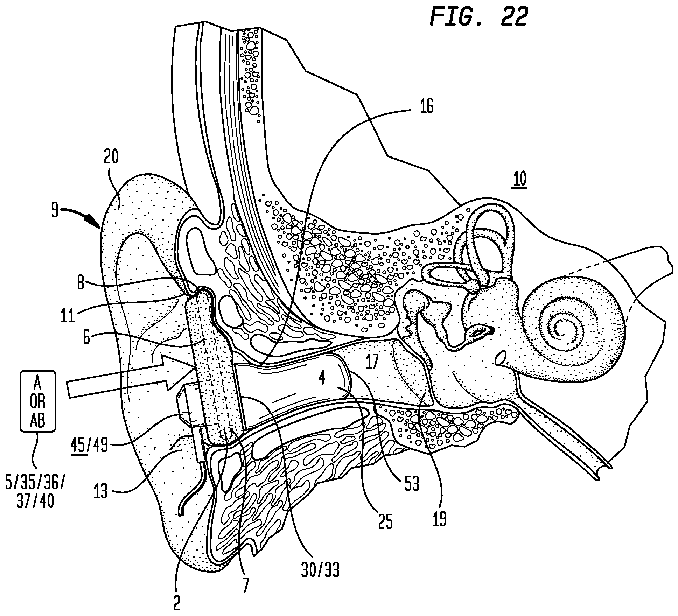

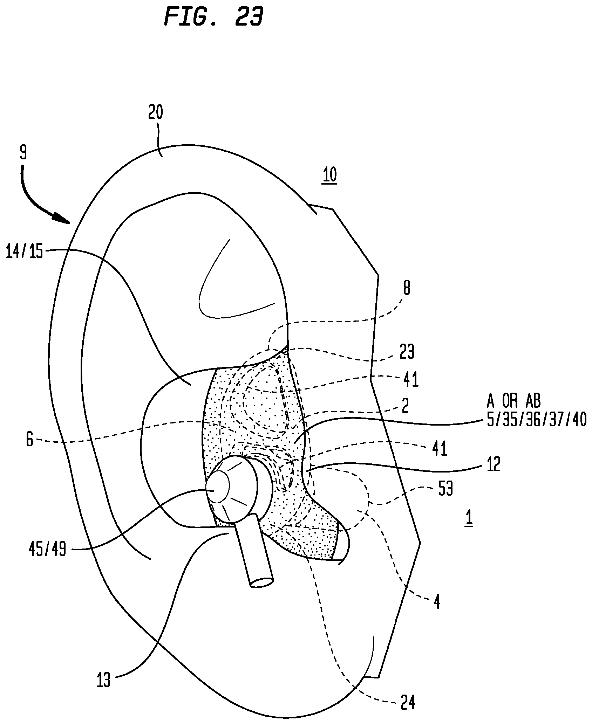

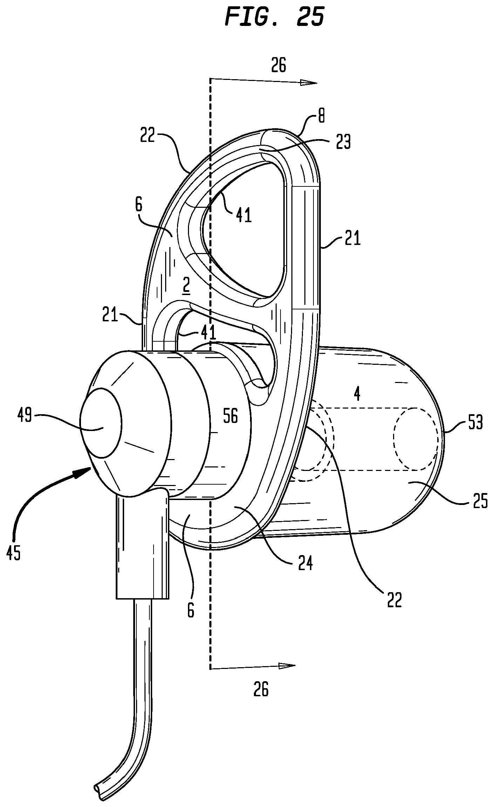

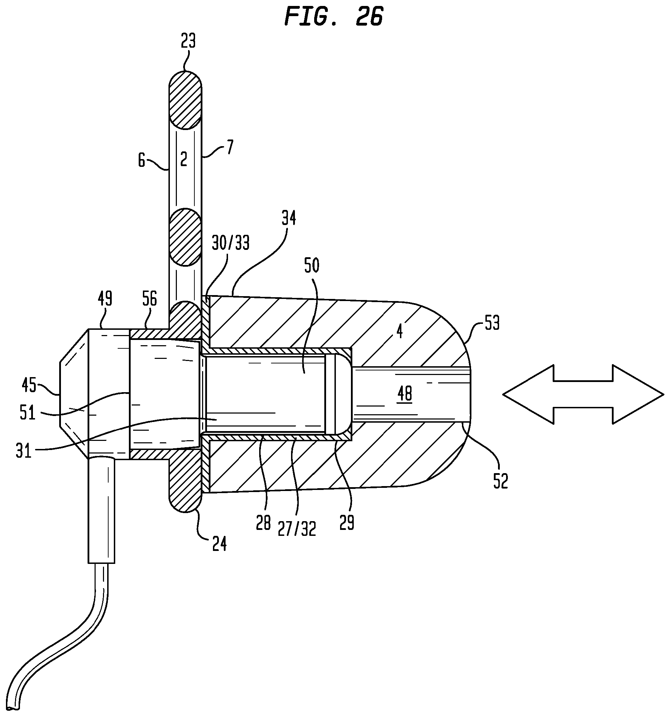

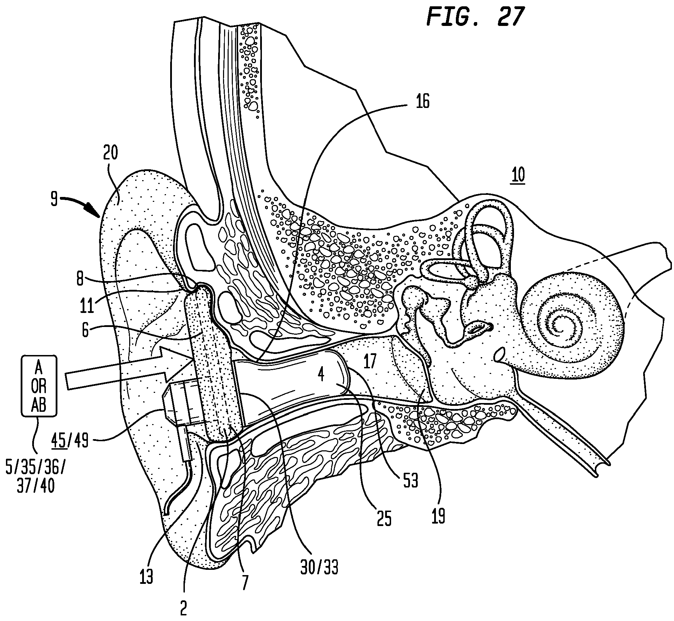

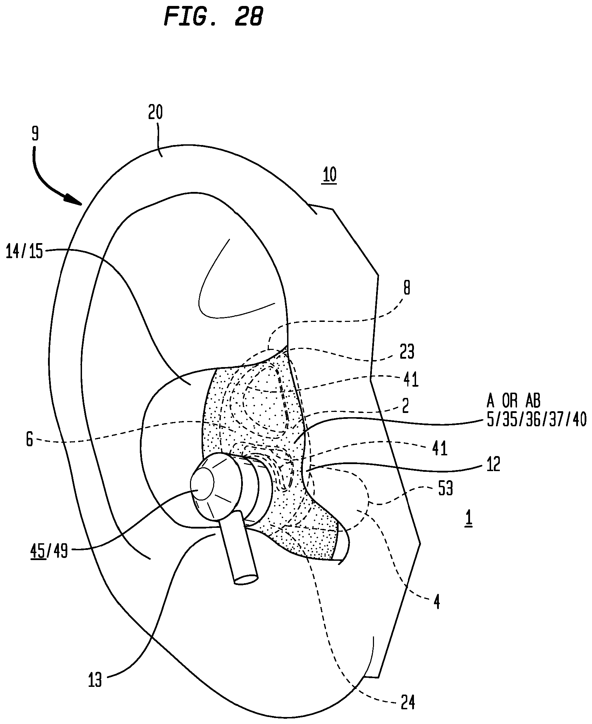

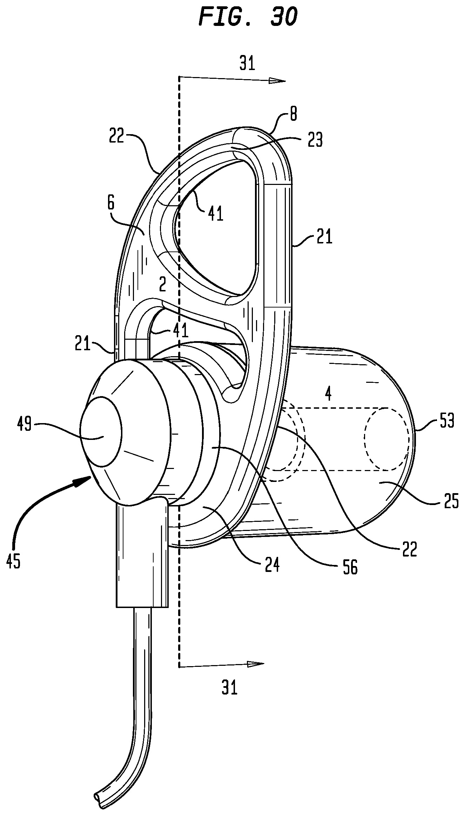

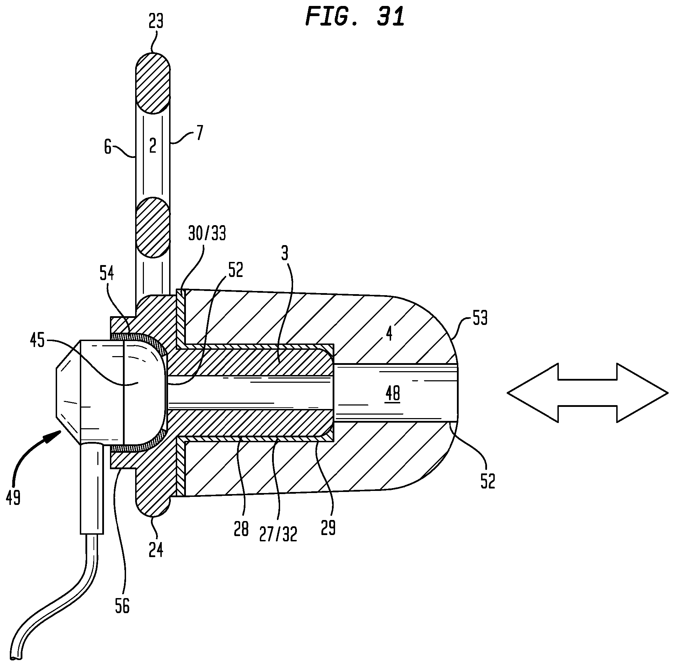

Generally referring to Figures 1 through 31, an earpiece (1) includes: an intra-auricular support (2), a projection element (3), a flexible body (4), and a moldable earpiece material (5). The intra-auricular support (2) has an outer surface (6) opposite an inner surface (7), where each surface extends to an intra-auricular support peripheral edge (8). The intra-auricular support (2) extending to the intra-auricular support peripheral edge (8) is configured to be positioned within an auricle (9) of the ear (10). As to particular embodiments, the intra-auricular support (2) is positioned within the area of the auricle (9) of the ear (10) anatomically defined by the antihelix (11), tragus (12), antitragus (13), and concha (14), also referred to herein as the concha bowl (15), and concurrently defining an inner surface (7) of the intra-auricular support (2) of sufficient area which precludes ingress of the external ear canal opening (16)(as shown in the example of Figure 7). For purposes of this invention the term "external ear canal (17)" means the portion of the ear canal (or auditory canal) communicating between the auricle (9) of the ear (10) and the tympanic membrane (19). For purposes of this invention, the term, "auricle (9)" means the area of the ear (10) outside of the external ear canal (17) including the concha bowl (15) and extending to the peripheral edge (20) of the ear (10)(also referred to as the "car pinna").

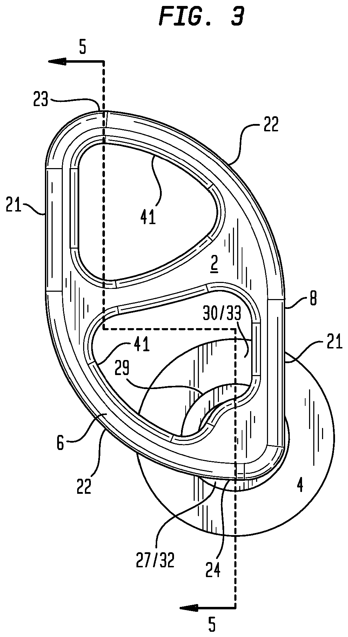

Now referring primarily to Figures 3 and 7, as to particular embodiments, the intra-auricular support (2) is configured to be positioned in the concha bowl (15) concurrent with the flexible body (4) engaged with the external ear canal (17). While the intra-auricular support peripheral edge (8) of the embodiment shown in Figures 3 and 7 generally defines a quadrilateral having a pair of linear opposed sides (21) interconnected by a pair of arcuate sides (22), this example of the configuration of the intra-auricular support (2) is not intended to preclude other configurations of the intra-auricular support (2) in which the intra-auricular support peripheral edge (8) can generally define a circle, an ellipse, triangle, or other configuration disposable in the auricle (9) of the ear (10) and, more particularly, disposed within the concha bowl (15).

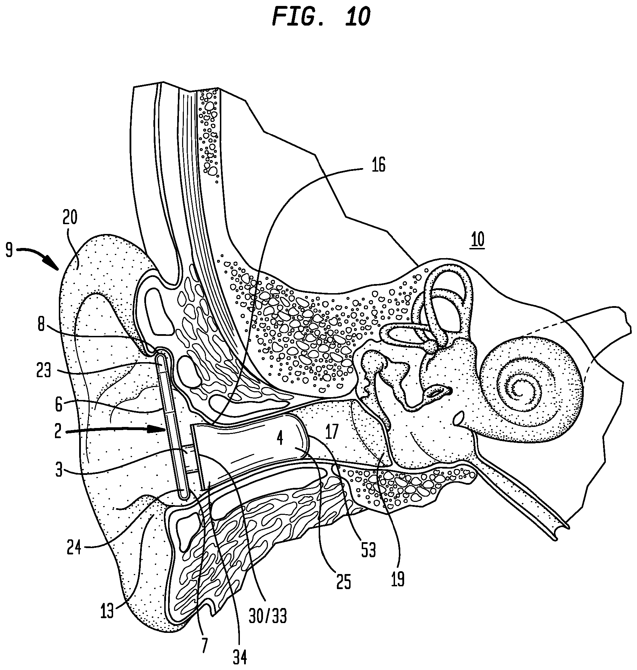

Now referring primarily to Figures 7 and 10, the intra-auricular support (2) is configured to be disposed in the concha bowl (15) and retained in the concha bowl (15) by contact of the antihelix (11), antitragus (13), or tragus (12) with the corresponding portions of the intra-auricular support (2). As shown by the example of Figure 7, a top portion (23) of the intra-auricular support peripheral edge (8) can be disposed behind the antihelix (11), and the lower portion (24) of the intra-auricular support peripheral edge (8) can be disposed behind the antitragus (13) and tragus (12).

Embodiments of the intra-auricular support (2) can fabricated, formed or molded from a wide variety of rigid or elastic thermoplastics including, but not necessarily limited to, acrylic, nylon, acrylonitrile butadiene styrene, polylactic acid, polybenzimidzole, polycarbonate, polyether sulfone, polyethylene, urethane, silicone, or combinations thereof.

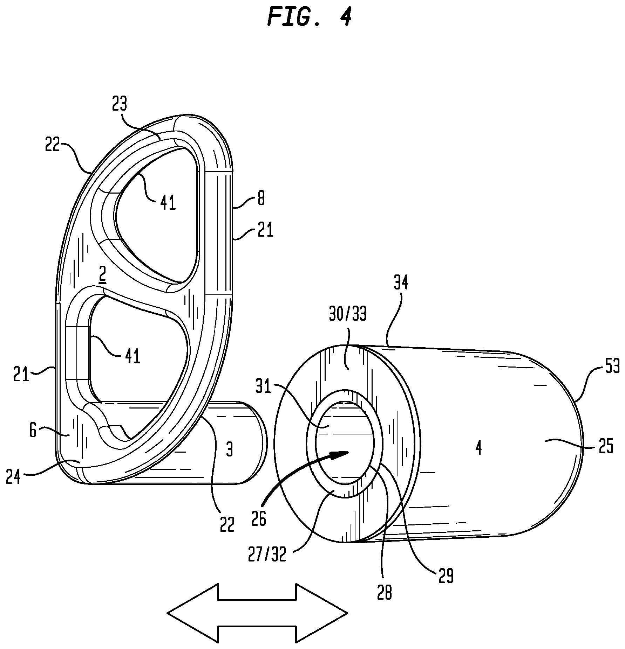

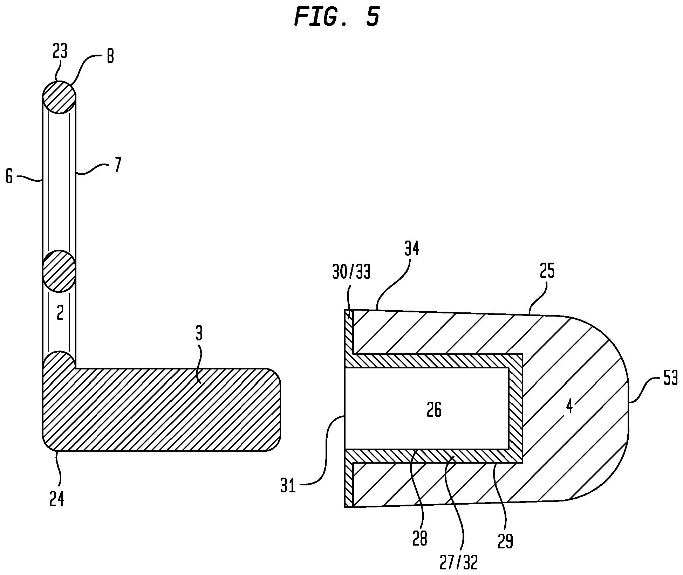

Now referring primarily to Figures 4 and 5, the earpiece (1) includes a projection element (3) outwardly extending from a location on the inner surface (7) of the intra-auricular support (2) which aligns the projection element (3) within the external ear canal (17) concurrent with the intra-auricular support (2) being disposed within the auricle (9) or concha bowl (15) of the ear (10). The projection element (3) is disposed on, coupled to, or directly coupled to the inner surface (7) of the intra-auricular support (2) at a location where the projection element (3) extends into the external ear canal (17) concurrent with the intra-auricular support (2) being disposed within the concha bowl (15) locating the inner surface (7) adjacent to the external ear canal opening (16). As exemplified in Figures 4 and 5, the location from which the projection element (3) can extend, the bottom portion of inner surface (7) of the intra-auricular support (2), can be proximate the intra-auricular support peripheral edge (8). The projection element (3) can be configured as a solid or tubular elongate member having circular, oval, square, rectangular, or cross sectional configuration otherwise disposable in the external ear canal (17).

The projection element (3) can comprise the same material(s) or different material(s) as the intra-auricular support (2), and as to particular embodiments, the intra-auricular support (2) and the projection element (3) can, but need not necessarily, be fabricated, formed or molded in one piece.

Again, referring generally to Figures 1 through 5 and Figures 10 through 31, the earpiece (1) includes a flexible body (4) disposed about the projection element (3). The flexible body (4) can be disposed inside of the external ear canal (17) of an ear (10) concurrent with the intra-auricular support (2) engaged within the auricle (9) or the concha bowl (15) with the inner surface (7) adjacent the external ear canal opening (16). Embodiments of the flexible body (4) can, but need not necessarily, comprise any one or a combination of pliant materials or bladders which deformably compress upon engagement with the external ear canal (17) and return toward the original configuration upon disengagement with surfaces of the external ear canal (17), such as foam, foam rubber, or hydrogel. As to particular embodiments, the flexible body (4) can further include a continuously smooth external surface (25).

As to particular embodiments, the flexible body (4) can, but need not necessarily, be integrated with the projection element (3) as one piece or, as to other embodiments, removably coupled or connected to the projection element (3), as illustrated in the example of Figure 4. In particular embodiments where the flexible body (4) removably couples to the projection element (3), the flexible body (4) can include a passage (26) disposed in the flexible body (4) and configured to accept the projection element (3). As illustrated in Figure 5, the projection element (3) can be configured as a solid cylinder which can insertingly disposed in the flexible body passage (26) of the flexible body (4). As to particular embodiments, the flexible body (4) can further include a flexible body conduit (27) having a flexible body conduit internal surface (28) which defines the flexible body passage (26) and an external surface (29) which engages the flexible body (4). The flexible body conduit (27) can further include an annular flange (30) extending radially outward from the passage first end (31) toward the external surface (29) of the flexible body (4). As to particular embodiments, the flexible body conduit (27) and the annular flange (30) can comprise one piece. In other particular embodiments, as illustrated in Figures 1 and 3, the flexible body conduit (27) can, but need not necessarily, comprise two components where the first component (32) comprises the flexible body conduit (27) disposed within the passage (26) of the flexible body (4), and the second component (33) comprises the annular flange (30) coupled to the flexible body first end (34) and radially extending outward from the first component (32) disposed in the passage (26) toward the external surface (29) of the flexible body (4).

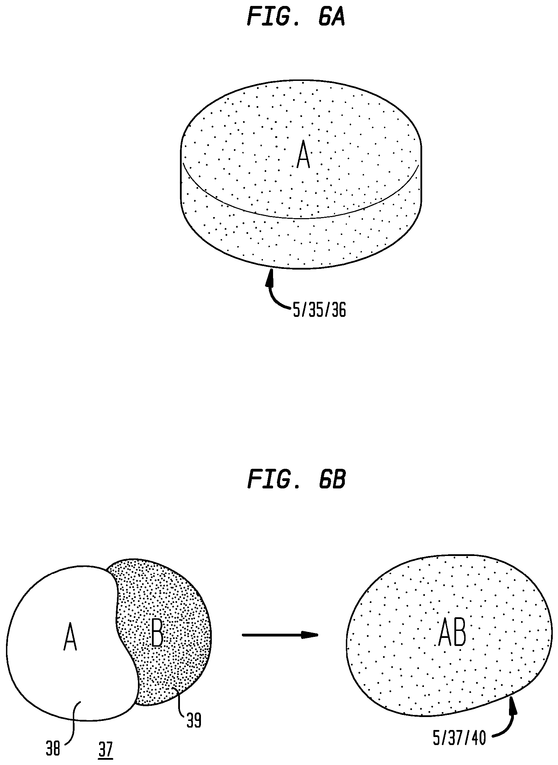

Now referring primarily to Figures 6A and 6B, and 11 through 13, 17, 18, 22, 23, 27 and the earpiece (1) includes a moldable earpiece material (5). The moldable earpiece material (5) is molded about the intra-auricular support (2) to conform to the auricle (9) or the concha bowl (15) of the ear (10). The moldable earpiece material (5) can, but need not necessarily be, a single compound (35) (shown as compound "A" in Figure 6A).

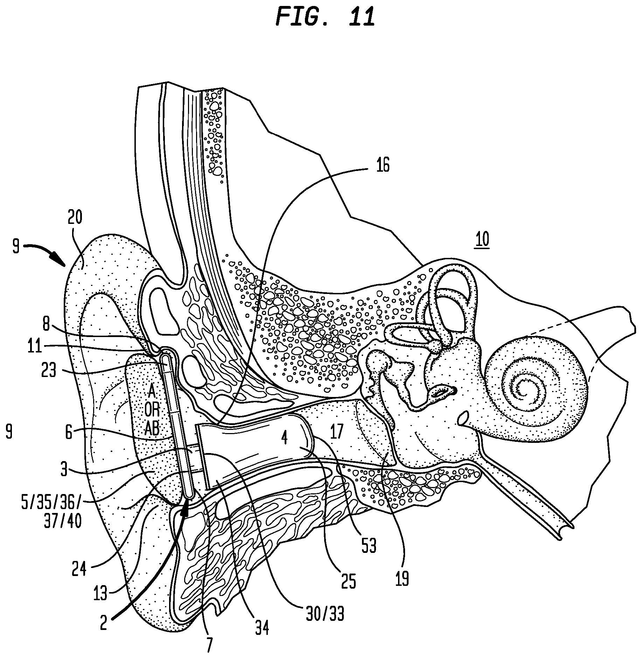

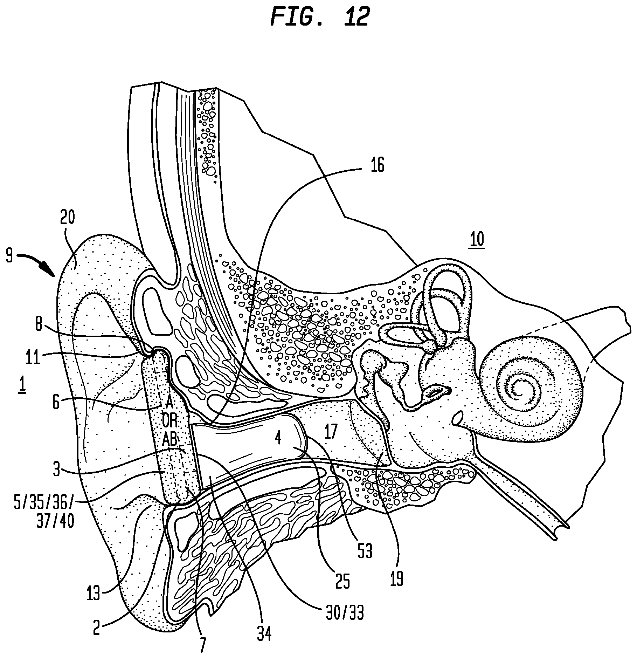

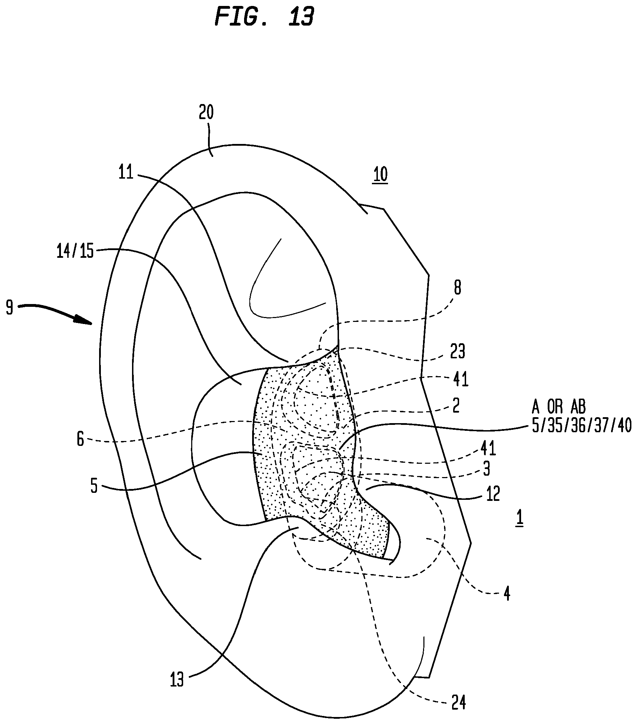

As to other embodiments, the moldable earpiece material (5) can, but need not necessarily, comprise a two-part moldable earpiece material (37) including or consisting of a moldable agent (38) (shown as compound "A" in Figure 6B) combinable with a curing agent (39) (shown as compound "B" in Figure 6B) which can be mixed together prior to molding to form a single mixed compound (40) (shown as compound "AB" in Figure 6B) capable of being moldable about, coupled to, or adhering to the intra-auricular support (2) by forcibly urging the single mixed compound (40) onto or about the intra-auricular support (2) (as shown in the example of Figures 11 and 12, 17 and 18, 22 and 23, and 27 and 28). Concurrently, the moldable earpiece material (5), whether a one-part moldable earpiece material (36) or a two-part moldable earpiece material (37) to provide a single mixed compound (40), is molded to the contour of the auricle (9) or the concha bowl (15) of the ear (10) (as shown in the example of Figures 12 and 13, 17 and 18, and 22 and 23). After molding about the intra-auricular support (2), the single compound (35) or single mixed compound (40) can then cure over a period of time at ambient temperature (or at a temperature greater or lesser than ambient temperature) to provide a fixed configuration of the moldable earpiece material (5)(as shown in the examples of Figures 13, 18, and 23).

In embodiments including a moldable earpiece material (5) comprising or consisting of a single compound (35), the single compound (35) can include or consist of one or more of: ethyl vinyl acetate, polycaprolactone, silicone, or like thermoplastic materials. Single compound (35) moldable earpiece materials (5) transition from a moldable condition to the fixed condition by exposure to external factors such as moisture, temperature, or ultraviolet light.

As one illustrative example, the single compound (35) can be an amount of polycaprolactone polymer (CAS No.: 24989-40-4) having the properties described in Table 1; however, this illustrative example is not intended to preclude the use of other thermoplastic polymers suitable for use with embodiments of the earpiece (1).

**TABLE 1. Physical Properties of Polycaprolactone Thermoplastic Polymers**

| Physical Property | ASTM Test | | | |
|---|---|---|---|---|
| Molecular Weight | | | | |
| Mn | GPC, THF, 25°C | 37,000±2000; | 47500 ± 2000; | 69000 ± 1500 |
| Mw | GPC, THF, 25°C | | 84500 ±1000; | 120000 ± 2000 |
| Mz | GPC, THF, 25°C | | 130000 ± 5000; | 178500 |
| Polydispersity (Mw/Mn) | | | 1.78 | 1.74 |
| Melt Flow Index | D 1238 | | | |
| 80°C, 2.16kg, g/10 min | | | 2.36 | 0.59 |
| 80°C, 21.6kg, g/10 min | | | 34.6 | 9.56 |
| 190°C, 2.16kg, g/10 min | | | 28 | 7.29 |
| Thermal Analysis (DSC) | | | | |
| Melting Point °C | | | 60-62 | 60-62 |
| Heat Of Fusion, DHm, J/g | | | 76.9 | 76.6 |
| Crystallinity, % | | | 56 | 56 |
| Crystallisation Temperature, °C | | | 25.2 | 27.4 |
| Glass Transition Temperature, Tg ,°C | | | -60 | -60 |
| Tensile Properties | | | | |
| Yield Stress, s y, Mpa | D 412-87 | | | |
| 100mm/min | | | 17.5 | 16 |
| 500mm/min | | | 17.2 | 14 |
| Modulus , E. Mpa | D 412-87 | | | |
| 1mm/min | | | 470 | 440 |
| 10mm/min | | | 430 | 500 |
| Draw Stress ,s d, MPa | D 412-87 | | | |
| 100mm/min | | | 12.6 | 11.9 |
| 500mm/min | | | 11.5 | 11 |
| Draw Ratio, l d, x | D 412-87 | | | |
| 100mm/min | | | >4.2 | 4 |
| Stress At Break ,s b, Mpa | D 412-87 | | | |
| 100mm/min | | | 29 | 54 |
| Strain At Break, e b, % | | | | |
| 100mm/min | D 412-87 | | >700 | 920 |
| Flexural Modulus, E , MPa | | | | |
| 2mm/min | D 790 | | 411 | nd |
| Hardness | D 2240 | | | |
| Shore A | | | 95 | 94 |
| Shore D | | | 51 | 50 |
| Viscosity | | | | |
| Pa. sec, 70°C ,10 1/sec | | | 2890 | 12650 |
| Pa. sec, 100°C ,10 1/sec | | | 1353 | 5780 |
| Pa. sec, 150°C ,10 1/sec | | | 443 | 1925 |

Polycaprolactone polymers can be heated to achieve a moldable condition, molded about the intra-auricular support (2), and contoured to the configuration of the auricle (9) or concha bowl (15) and cooled to achieve the fixed configuration of the earpiece (1).

Polycaprolactone polymers impart good water, oil, solvent, and chlorine resistance. Polycaprolactone polymers are also compatible with a wide range of other materials (collectively referred to as "admixed agents"), such as: starch, to impart greater biodegradability; colorants, such as alcohol dyes or acrylic coloring agents; powders, such as acrylic powder; particulates of plastic, copolymer plastics, metal, bismuth oxychloride, or glitter, or the like, either separately or in various combinations. Polycaprolactone polymers are non-toxic and approved by the United States Food and Drug Administration for specific applications in the human body.

In embodiments including a moldable earpiece material (5) comprising or consisting of a single mixed compound (40), the single mixed compound (40) can include a crosslinkable polymer (compound "A") having at least one hydrolysable silane group, selected from the group including or consisting of: silane-modified polyoxyalkylenes, polyolefins, poly(meth)acrylates, polyurethanes, polyamides, and polysiloxanes; silicone putty partially hydrolyzed alkyl silicate, or combinations thereof, and a catalyst (compound "B") including: a metallic salt of an organic carboxylic acid catalyst in which the metal comprises or consists of one or more of a platinum, tin, copper, or other metal.

One illustrative example of a single mixed compound (40) including a moldable agent (38) and curing agent (39) comprises polydimethylsiloxane polymer and platinum (0)-1,3-divinyl-1,1,3,3-tetramethyldisiloxane curing agent (39). Another illustrative example of a single mixed compound (40) including a moldable agent (38) and curing agent (39) comprises two proprietary compounds manufactured by Radians, Inc., silicone putty A-side and silicone putty B-side, of which one silicone putty contains methylpolysiloxanes.

Yet another illustrative example includes SILPURAN^{®} 8020, a platinum catalyst-curing solid silicone rubber available through Wacker Chemie AG.

**TABLE 2. Physical Properties of SILPURAN^{®} 8020**

| | |
|---|---|
| Hardness - Shore A (DIN 54615) | 62 |
| Cure System | Platinum (100:1.5 Base + catalyst) |
| Specific Gravity (DIN 54589 A) | 1.16 g/cm³ |
| Tensile Strength (DIN 54614S1) | 10.5 N/mm² |
| Elongation at break (DIN54614S1) | 751% |
| Rebound Resilience (DIN54622) | 58% |
| Tear Resistance (ASTM D624B) | 30 N/mm² |
| Comprssion set (22h/175°C)(DIN ISO815-B) | 30% |
| Appearance - Translucent | |

Now generally referring to Figures 1 through 31, embodiments of the intra-auricular support (2) can, but need not necessarily, include one or more aperture elements (41) which communicate between the outer surface (6) and the inner surface (7) of the intra-auricular support (2). As shown in the examples of Figures 11 and 12, the moldable earpiece material (5) can pass through the aperture elements (41) to envelop both the outer surface (6) and inner surface (7) of the intra-auricular support (2) and further allow the moldable earpiece material (5) to conform to the auricle (9) or concha bowl (15) of the ear (10). Additionally, by engaging the moldable earpiece material (5) with the one or more aperture elements (41), a greater surface area of the intra-auricular support (2) can be integrated with the moldable earpiece material (5) than if the moldable earpiece material (5) only engaged the outer surface (6), thereby making the cured moldable earpiece material (5) less likely to separate in whole or in part from the intra-auricular support (2).

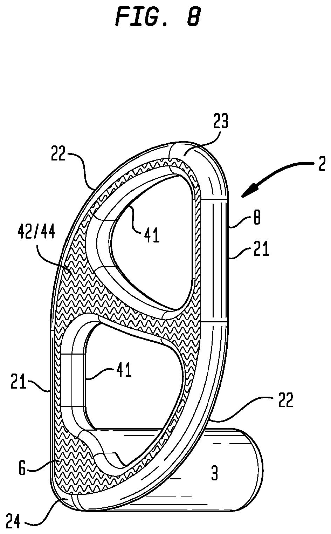

Now referring primarily to Figure 8, embodiments of the intra-auricular support (2) can, but need not necessarily, include adhesion elements (42). As to particular embodiments, the adhesion elements (42) can be configured as patterned surface elements (44) disposed on or in the outer surface (6) or inner surface (7) of the intra-articular support (2). The patterned surface elements (44) can provide an irregular or uniform pattern, texture, or roughness sufficient to fix or reduce travel of the moldable earpiece material (5) on or about the intra-auricular support (2). As to certain embodiments, the patterned surface elements (44) can provide recesses disposed in the intra-auricular support (2), which can function to sequester the moldable earpiece material (5) (as shown in Figure 8), or provide protuberances that extend outward of the intra-auricular support (2) to penetrate the moldable earpiece material (5), or combinations thereof. The patterned surface elements (44) can be one piece with the intra-auricular support (2) or can be applied to the intra-auricular support (2) as a layer or individual elements.

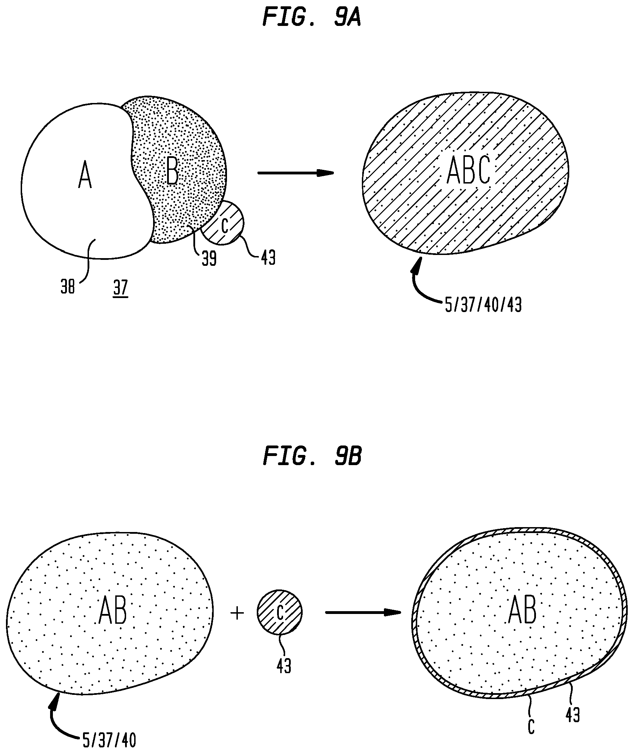

Now referring primarily to Figures 9A and 9B, as to particular embodiments of the intra-auricular support (2), the adhesion elements (42) can comprise a tackifier (43) (shown in Figures 9A and 9B as compound "C") incorporated into the moldable earpiece material (5) or applied to the inner surface (7) or the outer surface (6) of the intra-auricular support (2) to increase the tack, or stickiness, of the moldable earpiece material (5) or intra-auricular support (2). As illustrative examples, the tackifier (43) can include or consist of one or more of rosin resins, hydrocarbon resins, terpene resins, or other like materials.

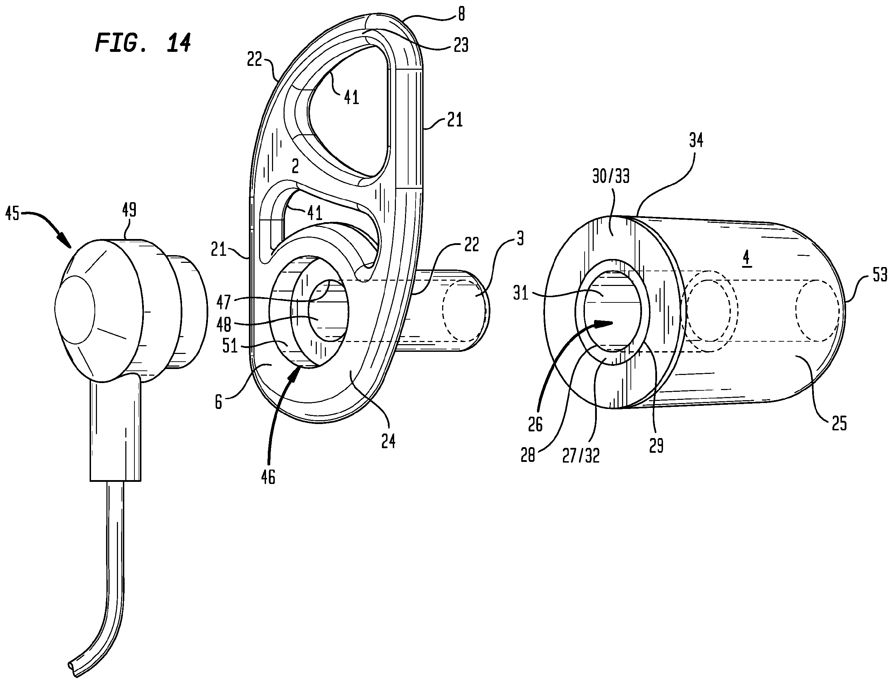

Now referring generally to Figures 14 through 31, particular embodiments of the earpiece (1) can be provided separate from, but capable of use with, one or more of a wide variety of in-ear devices (45), for example: earphones, earplugs, earbuds, sealing balloons, ear tips, ear tubes, ear speakers, and in-ear hearing aids, or the like. As to particular embodiments, the in-ear devices (45) can be a part of an apparatus worn or which resides outside of the ear (10), for example: headsets, head phones, telephones, BLUETOOTH^{®} headphones, wireless headphones, hearing aids, medical apparatus, or the like.

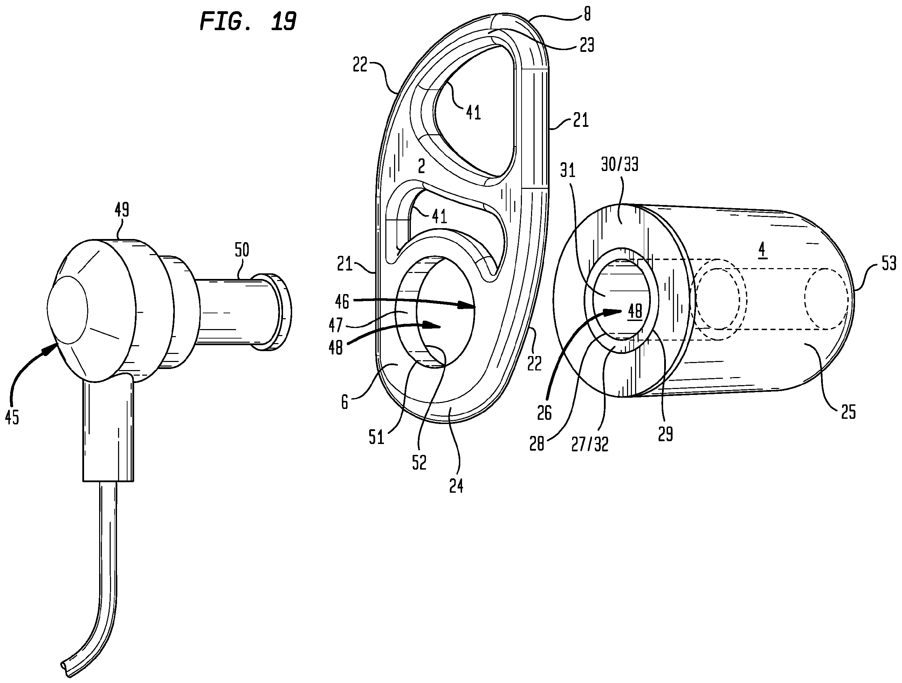

Particular embodiments of the earpiece (1) capable of use with an in-ear device (45) can include an intra-auricular support conduit (46) communicating between the outer surface (6) of the intra-auricular support (2) and the inner surface (7) of the intra-auricular support (2). The intra-auricular support conduit (46) can have an intra-auricular support conduit internal surface (47) configured to retain or releasably retain the in-ear device (45) in the earpiece (1) and which defines an intra-auricular support passage (48). The configuration of the intra-auricular support conduit internal surface (47) of the intra-auricular support conduit (46) can vary between embodiments to retain or releasably retain a wide variety of configurations of the in-ear device (45) including those configurations of the in-ear device (45) having a body (49) and a sound delivery element (50) which extends outward of the body (49) (as shown in the examples of Figures 19 through 28), or those configurations of the in-ear device (45) having a body (49) without a sound delivery element (50) which extends outward of the body (49) (as shown in the examples of Figures 14 through 18 and 29 through 31).

Now referring primarily to Figures 14 through 18, as to particular embodiments useful with in-ear devices (45) without an extended sound delivery element (50), the intra-auricular support (2) can include an intra-auricular support conduit (46) having an intra-auricular support conduit internal surface (47) communicating between an intra-auricular support conduit first end (51) open at the outer surface (6) of the intra-auricular support (2) and an intra-auricular support conduit second end (52) open at the flexible body second end (53) and defining an intra-auricular support passage (48). The intra-auricular support conduit (46) can be configured proximate the intra-auricular support conduit first end (51) to releasably retain the in-ear device (45). The configuration of the intra-auricular support conduit internal surface (47) can vary between embodiments to retain or releasably retain a wide variety of configurations of the in-ear device (45). The intra-auricular support conduit passage (48) communicates from the outer surface (6) of the intra-auricular support (2) to the inner surface (7) of the intra-auricular support (2), through the projection element (3), and through the flexible body (4) to the flexible body second end (53) for the transmission of sound from the in-ear device (45) releasably retained at the intra-auricular support conduit first end (51) to the flexible body second end (53).

Now referring generally to Figures 19 through 28, as to particular embodiments useful with in-ear device (45) including a sound delivery element (50) which projects outward of the body (49) of the in-ear device (45), the projection element (3) extending outward of the inner surface (7) of the intra-auricular support (2) can comprise or be substituted by the sound delivery element (50) of the in-ear device (45) and the flexible body (4) can be removably coupled to the sound delivery element (50) of the in-ear device (45). As to these embodiments, the passage (26) disposed in the flexible body (4) can be configured to insertingly receive, in part or the entirety, the sound delivery element (50) of in-ear device (45).

Now referring primarily to Figures 19 through 23, as to particular embodiments of the earpiece (1) which accommodate an in-ear device (45) having a sound delivery element (50) extending outward of the body (49), the an intra-auricular support conduit (46) can have an intra-auricular support conduit first end (51) disposed at the outer surface (6) of the intra-auricular support (2) and an intra-auricular support conduit second end (52) disposed at the inner surface (7) of the intra-auricular support (2). The intra-auricular support conduit internal surface (47) can be configured to releasably retain the body (49) of in-ear device (45) with the sound delivery element (50) extending outward of the inner surface (7) of the intra-auricular support (2). The intra-auricular support conduit passage (48) communicates from the outer surface (6) of the intra-auricular support (2) to the inner surface (7) of the intra-auricular support (2) and through the flexible body (4) to open at the flexible body second end (53) for the transmission of sound from the in-ear device (45) releasably retained at the intra-auricular support conduit first end (51) to the flexible body second end (53).

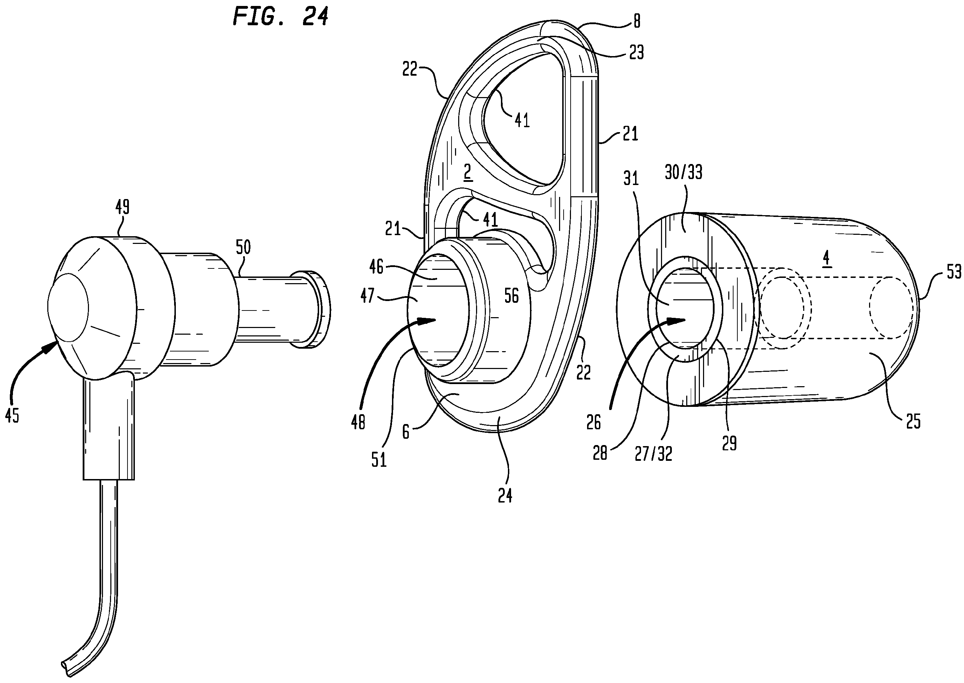

Now referring primarily to Figures 24 through 28, as to particular embodiments the intra-auricular support (2) can, but need not necessarily, include a tubular member (56) which extends a distance outward from the outer surface (6) of the intra-auricular support (2) to correspondingly dispose the intra-auricular support conduit first end (51) a distance outward of the outer surface (6) of the intra-auricular support (2). The intra-auricular support conduit internal surface (47) of the tubular member (56) can be configured to releasably retain the body (49) of the in-ear device (45). As to those in-ear devices (45) having a sound delivery element (50) extending a distance outward of the inner surface (7) of the intra-auricular support (2), the sound delivery element (50) extending outward of the intra-auricular support conduit internal surface (47) can be insertingly received in the flexible body (4) with the conduit passage (48) communicating from the outer surface (6) of the intra-auricular support (2) to the inner surface (7) of the intra-auricular support (2), through the flexible body (4) to open at the flexible body second end (53) for the transmission of sound from the in-ear device (45) releasably retained at the intra-auricular support conduit first end (51) to the flexible body second end (53).

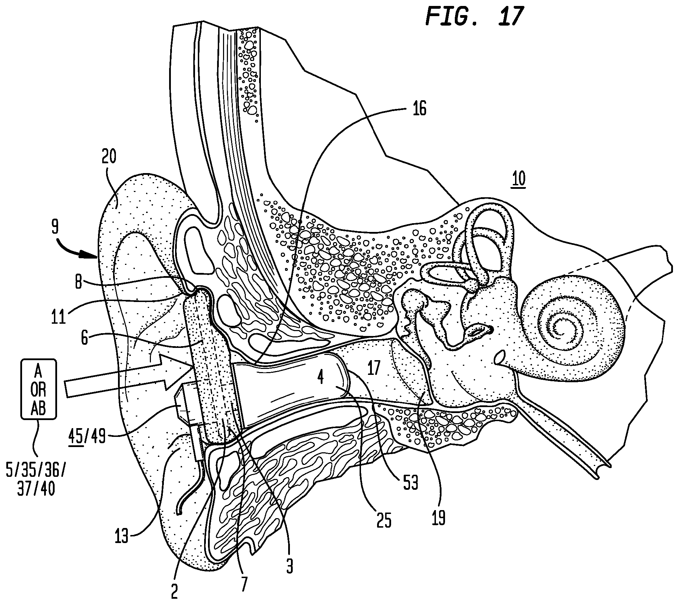

As described above and again exemplified in Figures 17, 18, 22, 23, 27, and 28, particular embodiments of the earpiece (1) including an in-ear device (45) can include a moldable earpiece material (5) moldable about the intra-auricular support (2) to conform to the auricle (9) of the ear (10). The configuration of the embodiment shown in Figures 24 through 28 confers the advantage of molding the moldable earpiece material (5) around the tubular member (56) extending outward of the outer surface (6) of the intra-auricular support (2), thereby avoiding or reducing ingress of the amount of moldable earpiece material (5) in the intra-auricular support conduit passage (48) or in contact with the in-ear device (45).

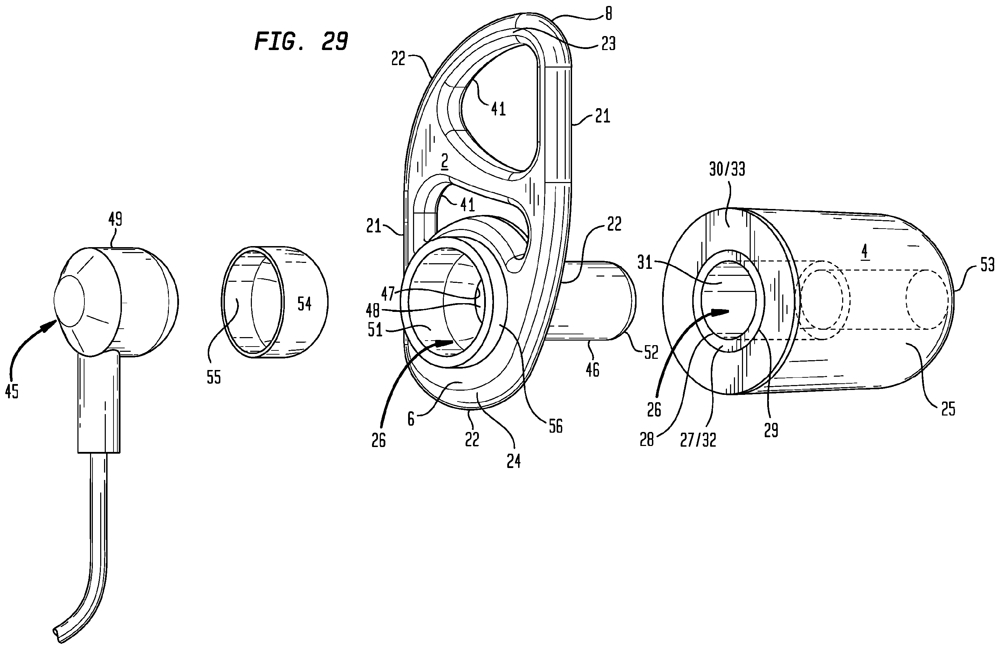

Now referring to Figures 29 through 31, embodiments can, but need not necessarily include, a flexible elastomer insert (54) which removably couples to the intra-auricular support conduit (46) proximate the intra-auricular support conduit first end (51) to provide a flexible elastomer insert internal surface (55) configured to retain or releasably retain the in-ear device (45) in the intra-auricular support (2) or the earpiece (1). A plurality of flexible elastomer inserts (54) can provide a plurality of different configurations of the flexible elastomer internal surface (55) to retain or releasably retain a corresponding plurality of different configurations of the body (49) of the in-ear device (45). By interchanging the flexible elastomer insert (54) removably coupled within the intra-auricular support conduit (46) a plurality of different configurations of the flexible elastomer internal surface (55) can be achieved in one configuration of the intra-auricular support conduit (46), whereby a corresponding plurality of in-ear devices (45) of different configuration can be releasably retained. While the illustrative flexible elastomer insert shown in Figures 29-31 has a particular thickness, this is not intended to preclude embodiments of greater or lesser thickness and the thickness of particular embodiments can be as great as necessary to retain the in-ear device (45) in the intra-auricular support conduit (46) or the earpiece (1).

Now referring generally to Figures 1 through 31 and, more particularly to Figures 10, 11, and 12, a method of using the earpiece (1) can include obtaining an earpiece (1) including: an intra-auricular support (2), a projection element (3), a flexible body (4) disposed about the projection element (3), and a moldable earpiece material (5), disposing the flexible body (4) in the external ear canal (17) of an ear (10), adjusting the intra-auricular support (2) within the auricle (9) or concha bowl (15) having the intra-auricular support inner surface (7) disposed substantially adjacent the external ear canal opening (16), pressingly engaging the moldable earpiece material (5) about the intra-auricular support (2), conforming the moldable earpiece material (5) to the auricle (9) or the concha bowl (15) of the ear (10), and curing the moldable earpiece material (5) to provide a fixed configuration of the earpiece (1); the intra-auricular support (2), projection element (3), flexible body (4), and moldable earpiece material (5) having been described above.

In particular embodiments, as exemplified in Figures 17, 18, 22, 23, 27, and 28, a method of using the earpiece (1) may further include obtaining an intra-auricular support conduit (46) having an intra-auricular support conduit (46) configured to releasably retain an in-ear device (45), as described above, and releasably retaining an in-ear device (45) to the intra-auricular support conduit (46). As to particular embodiments, the method can further include releasably retaining a body (49) of an in-ear device (45) to the intra-auricular support conduit (46) with a sound delivery element (50) extending a distance outward of the inner surface (7) of the intra-auricular support (2), and removably coupling a flexible body (4) about the sound delivery element (50).

As can be easily understood from the foregoing, the basic concepts of the present invention may be embodied in a variety of ways. The invention involves numerous and varied embodiments of an earpiece which can be utilized for the production of an earpiece by the process above described.

As such, the particular embodiments or elements of the invention disclosed by the description or shown in the figures or tables accompanying this application are not intended to be limiting, but rather exemplary of the numerous and varied embodiments generically encompassed by the invention or equivalents encompassed with respect to any particular element thereof. In addition, the specific description of a single embodiment or element of the invention may not explicitly describe all embodiments or elements possible; many alternatives are implicitly disclosed by the description and figures.

It should be understood that each element of an apparatus or each step of a method may be described by an apparatus term or method term. Such terms can be substituted where desired to make explicit the implicitly broad coverage to which this invention is entitled. As but one example, it should be understood that all steps of a method may be disclosed as an action, a means for taking that action, or as an element which causes that action. Similarly, each element of an apparatus may be disclosed as the physical element or the action which that physical element facilitates. As but one example, the disclosure of "a moldable earpiece material (5)" should be understood to encompass disclosure of the act of "molding an earpiece material" -- whether explicitly discussed or not -- and, conversely, were there effectively disclosure of the act of "molding an earpiece material", such a disclosure should be understood to encompass disclosure of "a moldable earpiece material (5)" and even a "means for molding an earpiece material." Such alternative terms for each element or step are to be understood to be explicitly included in the description.

In addition, as to each term used it should be understood that unless its utilization in this application is inconsistent with such interpretation, common dictionary definitions should be understood to be included in the description for each term as contained in the Random House Webster's Unabridged Dictionary, second edition.

Moreover, for the purposes of the present invention, the term "a" or "an" entity refers to one or more of that entity; for example, "an earpiece" refers to one or more of the earpieces. As such, the terms "a" or "an", "one or more" and "at least one" can be used interchangeably herein.

All numeric values herein are assumed to be modified by the term "about", whether or not explicitly indicated. For the purposes of the present invention, ranges may be expressed as from "about" one particular value to "about" another particular value. When such a range is expressed, another embodiment includes from the one particular value to the other particular value. The recitation of numerical ranges by endpoints includes all the numeric values subsumed within that range. A numerical range of one to five includes for example the numeric values 1, 1.5, 2, 2.75, 3, 3.80, 4, 5, and so forth. It will be further understood that the endpoints of each of the ranges are significant both in relation to the other endpoint, and independently of the other endpoint.

Similarly, the antecedent "substantially" means largely, but not wholly, the same form. manner or degree and the particular element will have a range of configurations as a person of ordinary skill in the art would consider as having the same function or result. When a particular element is expressed as an approximation by use of the antecedent "substantially," it will be understood that the particular element forms another embodiment.

## Claims

1. An earpiece (1), comprising:
an intra-auricular support (2) having an outer surface (6) opposite an inner surface (7) each extending to an intra-auricular support peripheral edge (8) and being configured to be positioned within the area of the auricle (9) of the wearer's ear (10) anatomically defined by the concha bowl (15), said intra-auricular support (2) being configured to engage said auricle (9) of the wearer's ear (10) and being configured to be retained in the concha bowl (15) by contact of the antihelix (11), antitragus (13), or tragus (12) with corresponding portions of the intra- auricular support (2);
a projection element (3) extending outward of said inner surface (7) of said intra-auricular support (2), wherein the projection element (3) is directly coupled to the inner surface (7) of the intra-auricular support (2) at a location where the projection element (3) extends into the external ear canal (17) concurrent with the intra-auricular support (2) being disposed within the concha bowl (15) locating the inner surface (7) adjacent to the external ear canal opening (16);
a flexible body (4) disposed about said projection element (3), said flexible body is configured to deformably compress upon engagement with said external ear canal (17) and return toward the original configuration upon disengagement with surfaces of the external ear canal (17), said flexible body (4) disposable inside of said external ear canal of said ear concurrent with said intra-auricular support (2) engaged to said auricle (9) of said ear; and
a moldable earpiece material (5) molded about said intra-auricular support (2) and to the contour of the auricle or the concha bowl of the wearer's ear to conform to said auricle (9) or to said concha bowl of said wearer's ear, said molded earpiece material (5) curable in a fixed configuration of said earpiece (1).

2. An earpiece (1), comprising:
an intra-auricular support (2) having an outer surface (6) opposite an inner surface (7) each extending to an intra-auricular support peripheral edge (8) and being configured to be positioned within the area of the auricle (9) of the wearer's ear (10) anatomically defined by the concha bowl (15), said intra-auricular support (2) being configured to engage said auricle (9) of the wearer's ear (10) and being configured to be retained in the concha bowl (15) by contact of the antihelix (11), antitragus (13), or tragus (12) with corresponding portions of the intra- auricular support (2);
an intra-auricular support conduit (46) disposed between said inner surface (7) and said outer surface (6) of said intra-auricular support (2), said intra-auricular support conduit (46) having a support conduit internal surface (47) configured to releasable retain an in-ear device; and
a moldable earpiece material moldable about said intra-auricular support (2), and to the contour of the auricle or the concha bowl of the wearer's ear and optionally about said intra-auricular support conduit (46) to conform to said auricle (9) or to said concha bowl of said wearer's ear , said moldable earpiece material (5) curable in a fixed configuration of said earpiece (1).

3. The earpiece (1) of claim 2, further comprising:
a projection element (3) extending outward of said inner surface (7) of said intra-auricular support (2); and
a flexible body (4) disposed about said projection element (3), said flexible body (4) disposable inside of an external ear canal of an ear concurrent with said intra-auricular support (2) engaged to an auricle (9) of said ear.

4. The earpiece (1) of claim 2, wherein said intra-auricular conduit (46) extends a distance outward of said outer surface (6) of said intra-auricular support (2).

5. The earpiece (1) of any one of claims 2, 3, 4, further comprising an in-ear device releasably retained by said intra-auricular support conduit (46).

6. The earpiece (1) of any one of claims 1 or 2, wherein said moldable earpiece material (5) comprises a one-part moldable earpiece material.

7. The earpiece (1) of claim 6, wherein said one-part moldable earpiece material is selected from the group consisting of: ethyl vinyl acetate, polycaprolactone, and room temperature vulcanization silicone.

8. The earpiece (1) of claim 7, wherein said one-part moldable earpiece material heated to achieve a moldable condition to conform to said auricle (9) of said ear in said fixed configuration of said earpiece.

9. The earpiece (1) of claim 8, wherein said one-part moldable earpiece material comprises an amount of polycaprolactone polymer having a number average molecular weight of about 37,000 grams per mole to 80,000 grams per mole.

10. The earpiece (1) of any one of claims 1 or 2, wherein said moldable earpiece material (5) comprises a moldable agent and a curing agent, wherein combination of said moldable agent and curing agent curable in said fixed configuration of said earpiece.

11. The earpiece (1) of claim 10, wherein said moldable agent and said curing agent comprises a crosslinkable polymer and a catalyst.

12. The earpiece (1) of claim 11, wherein said crosslinkable polymer comprises a polymer containing at least one hydrolysable silane group and wherein said catalyst comprises one or more of a metallic salt of an organic carboxylic acid.

13. The earpiece (1) of claim 11, wherein said crosslinkable polymer comprises one or more of silane-modified polyoxyalkylenes, polyolefins, poly(meth)acrylates, polyurethanes, polyamides, and polysiloxanes and said a metallic salt of an organic carboxylic acid comprises one or more of a platinum, tin or copper salt of said organic carboxylic acid.

14. The earpiece (1) of claim 11, wherein said crosslinkable polymer comprises polydimethylsiloxane and wherein said catalyst comprises platinum (0)-1,3-divinyl-1,1,3,3-tetramethyldisiloxane.

15. The earpiece (1) of any one of claims 1 or 2, further comprising one or more aperture elements communicating between said outer surface (6) and said inner surface (7) of said intra-auricular support (2).

16. The earpiece (1) of any one of claims 1 or 2, further comprising adhesion elements disposed on said intra-auricular support (2), said adhesion elements configured to increase adherence of said moldable earpiece material (5) to said intra-auricular support (2).

## Patentansprüche

1. Ohrteil (1), umfassend:
ein intraaurikuläres Halteteil (2), das eine Außenfläche (6) gegenüber einer Innenfläche (7), die sich jeweils zu einem Intraaurikuläres-Halteteil-Rand (8) erstrecken, aufweist und konfiguriert ist, um in dem anatomisch durch die Ohrschale (15) definierten Bereich der Ohrmuschel (9) des Ohrs (10) eines Trägers positioniert zu werden, wobei das intraaurikuläre Halteteil (2) konfiguriert ist, um in die Ohrmuschel (9) des Ohrs (10) des Trägers einzugreifen, und konfiguriert ist, um durch einen Kontakt der Antihelix (11), des Antitragus (13) oder des Tragus (12) mit entsprechenden Teilen des intraaurikulären Halteteils (2) in der Ohrschale (15) gehalten zu werden,
ein Vorsprungselement (3), das sich von der Innenfläche (7) des intraaurikulären Halteteils (2) nach außen erstreckt, wobei das Vorsprungselement (3) direkt mit der Innenfläche (7) des intraaurikulären Halteteils (2) an einer Position gekoppelt ist, an welcher sich das Vorsprungselement (3) in den Außenohrkanal (17) erstreckt, wenn das intraaurikuläre Halteteil (2) in der Ohrschale (15) angeordnet ist, sodass die Innenfläche (7) in Nachbarschaft zu der Außenohrkanalöffnung (16) positioniert wird,
einen flexiblen Körper (4), der um das Vorsprungselement (3) herum angeordnet ist, wobei der flexible Körper konfiguriert ist, um verformbar komprimiert zu werden, wenn er mit dem Außenohrkanal (17) eingreift, und zu der ursprünglichen Konfiguration zurückzukehren, wenn er von den Oberflächen des Außenohrkanals (17) gelöst wird, wobei der flexible Körper (4) in dem Außenohrkanal des Ohrs angeordnet werden kann, wenn das intraaurikuläre Halteteil (2) mit der Ohrmuschel (9) des Ohrs eingreift, und
ein formbares Ohrteilmaterial (5), das um das intraaurikuläre Halteteil (2) herum und an die Kontur der Ohrmuschel oder Ohrschale des Ohrs des Trägers geformt wird, um der Ohrmuschel (9) oder der Ohrschale des Ohrs des Trägers zu entsprechen, wobei das formbare Ohrteilmaterial (5) in einer fixierten Konfiguration des Ohrteils (1) ausgehärtet werden kann.

2. Ohrteil (1), umfassend:
ein intraaurikuläres Halteteil (2), das eine Außenfläche (6) gegenüber einer Innenfläche (7), die sich jeweils zu einem Intraaurikuläres-Halteteil-Rand (8) erstrecken, aufweist und konfiguriert ist, um in dem anatomisch durch die Ohrschale (15) definierten Bereich der Ohrmuschel (9) des Ohrs (10) eines Trägers positioniert zu werden, wobei das intraaurikuläre Halteteil (2) konfiguriert ist, um in die Ohrmuschel (9) des Ohrs (10) des Trägers einzugreifen, und konfiguriert ist, um durch einen Kontakt der Antihelix (11), des Antitragus (13) oder des Tragus (12) mit entsprechenden Teilen des intraaurikulären Halteteils (2) in der Ohrschale (15) gehalten zu werden,
eine Intraaurikuläres-Halteteil-Öffnung (46), die zwischen der Innenfläche (7) und der Außenfläche (6) des intraaurikulären Halteteils (2) angeordnet ist, wobei die Intraaurikuläres-Halteteil-Öffnung (46) eine Halteteilöffnung-Innenfläche (47), die für das lösbare Halten eines In-Ohr-Geräts konfiguriert ist, aufweist, und
ein formbares Ohrteilmaterial, das um das intraaurikuläre Halteteil (2) herum und an die Kontur der Ohrmuschel oder Ohrschale des Ohrs des Trägers und optional um die Intraaurikuläres-Halteteil-Öffnung (46) herum geformt wird, um der Ohrmuschel (9) oder der Ohrschale des Ohrs des Trägers zu entsprechen, wobei das formbare Ohrteilmaterial (5) in einer fixierten Konfiguration des Ohrteils (1) ausgehärtet werden kann.

3. Ohrteil (1) nach Anspruch 2, das weiterhin umfasst:
ein Vorsprungselement (3), das sich von der Innenfläche (7) des intraaurikulären Halteteils (2) nach außen erstreckt, und
einen flexiblen Körper (4), der um das Vorsprungselement (3) herum angeordnet ist, wobei der flexible Körper (4) in einem Außenohrkanal eines Ohrs angeordnet werden kann, wenn das intraaurikuläre Halteteil (2) mit einer Ohrmuschel (9) des Ohrs eingreift.

4. Ohrteil (1) nach Anspruch 2, wobei sich die Intraaurikuläres-Halteteil-Öffnung (46) über eine Distanz von der Außenfläche (6) des intraaurikulären Halteteils (2) nach außen erstreckt.

5. Ohrteil (1) nach einem der Ansprüche 2, 3, 4, das weiterhin ein In-Ohr-Gerät, das lösbar durch die Intraaurikuläres-Halteteil-Öffnung (46) gehalten wird, umfasst.

6. Ohrteil (1) nach Anspruch 1 oder 2, wobei das formbare Ohrteilmaterial (5) ein einstückiges formbares Ohrteilmaterial umfasst.

7. Ohrteil (1) nach Anspruch 6, wobei das einstückige formbare Ohrteilmaterial aus der Gruppe ausgewählt wird, die aus Ethylvinylacetat, Polycaproacton und raumtemperaturvernetzendem Silikon besteht.

8. Ohrteil (1) nach Anspruch 7, wobei das einstückige formbare Ohrteilmaterial erhitzt wird, um einen formbaren Zustand für eine Anpassung an die Ohrmuschel (9) des Ohrs in der fixierten Konfiguration des Ohrteils zu erhalten.

9. Ohrteil (1) nach Anspruch 8, wobei das einstückige formbare Ohrteilmaterial eine Menge von Polycaprolacton-Polymer mit einem durchschnittlichen Molekulargewicht von ungefähr 37.000 Gramm pro Mol bis 80.000 Gramm pro Mol umfasst.

10. Ohrteil (1) nach Anspruch 1 oder 2, wobei das formbare Ohrteilmaterial (5) ein formbares Mittel und ein Aushärtungsmittel umfasst, wobei die Kombination aus dem formbaren Mittel und dem Aushärtungsmittel in der fixierten Konfiguration des Ohrteils ausgehärtet werden kann.

11. Ohrteil (1) nach Anspruch 10, wobei das formbare Mittel und das Aushärtungsmittel ein vernetzbares Polymer und einen Katalysator umfassen.

12. Ohrteil (1) nach Anspruch 11, wobei das vernetzbare Polymer ein Polymer, das wenigstens eine hydrolysierbare Silangruppe enthält, umfasst und wobei der Katalysator ein Metallsalz und/oder eine organische Carbonsäure umfasst.

13. Ohrteil (1) nach Anspruch 11, wobei das vernetzbare Polymer silanmodifizierte Polyoxyalkylene, Pololefine, Poly(meth)acrylate, Polurethane, Polyamide und/oder Polysiloxane umfasst und das Metallsalz einer organischen Carbonsäure ein Platin-, Zinn- oder Kupfersalz der organischen Carbonsäure umfasst.

14. Ohrteil (1) nach Anspruch 11, wobei das vernetzbare Polymer Polydimethylsiloxan umfasst und wobei der Katalysator Platin (0)-1,3-divinyl-1,1,3,3-tetramethyldisiloxan umfasst.

15. Ohrteil (1) nach Anspruch 1 oder 2, das weiterhin ein oder mehrere Öffnungselemente umfasst, die eine Verbindung zwischen der Außenfläche (6) und der Innenfläche (7) des intraaurikulären Halteteils (2) herstellen.

16. Ohrteil (1) nach Anspruch 1 oder 2, das weiterhin Haftungselemente umfasst, die an dem intraaurikulären Halteteil (2) angeordnet sind, wobei die Haftungselemente konfiguriert sind, um eine Haftung des formbaren Ohrteilmaterials (5) an dem intraaurikulären Halteteil (2) zu erhöhen.

## Revendications

1. Écouteur (1), comprenant :
un support intra-auriculaire (2) ayant une surface externe (6) opposée à une surface interne (7), chacune s'étendant jusqu'à un bord périphérique de support intra-auriculaire (8) et étant conçue pour être positionnée au sein de la zone du pavillon (9) de l'oreille du porteur (10) définie anatomiquement par l'arrondi de la conque (15), ledit support intra-auriculaire (2) étant conçu pour venir en prise avec ledit pavillon (9) de l'oreille du porteur (10) et étant conçu pour être retenu dans l'arrondi de la conque (15) par contact de l'anti-hélix (11), de l'anti-tragus (13) ou du tragus (12) avec les parties correspondantes du support intra-auriculaire (2) ;
un élément de saillie (3) s'étendant vers l'extérieur de ladite surface interne (7) dudit support intra-auriculaire (2), dans lequel l'élément de saillie (3) est directement couplé à la surface interne (7) du support intra-auriculaire (2) au niveau d'un emplacement où l'élément de saillie (3) s'étend dans le conduit auditif externe (17) en même temps que le support intra-auriculaire (2) est disposé au sein de l'arrondi de la conque (15) pour positionner la surface interne (7) de manière adjacente à l'ouverture du conduit auditif externe (16) ;
un corps souple (4) disposé autour dudit élément de saillie (3), ledit corps souple étant conçu pour se comprimer de manière déformable en cas de prise avec ledit conduit auditif externe (17) et revenir à la configuration d'origine en cas de dégagement d'avec les surfaces du conduit auditif externe (17), ledit corps souple (4) pouvant être disposé à l'intérieur dudit conduit auditif externe de ladite oreille en même temps que ledit support intra-auriculaire (2) est en prise avec ledit pavillon (9) de ladite oreille ; et
un matériau d'écouteur (5) pouvant être moulé qui est moulé autour dudit support intra-auriculaire (2) et sur le contour du pavillon ou de l'arrondi de la conque de ladite oreille du porteur pour se conformer audit pavillon (9) ou audit arrondi de la conque de ladite oreille du porteur, ledit matériau d'écouteur (5) moulé pouvant être durci dans une configuration fixe dudit écouteur (1).

2. Écouteur (1), comprenant :
un support intra-auriculaire (2) ayant une surface externe (6) opposée à une surface interne (7), chacune s'étendant jusqu'à un bord périphérique de support intra-auriculaire (8) et étant conçue pour être positionnée au sein de la zone du pavillon (9) de l'oreille du porteur (10) définie anatomiquement par l'arrondi de la conque (15), ledit support intra-auriculaire (2) étant conçu pour venir en prise avec ledit pavillon (9) de l'oreille du porteur (10) et étant conçu pour être retenu dans l'arrondi de la conque (15) par contact de l'anti-hélix (11), de l'anti-tragus (13) ou du tragus (12) avec les parties correspondantes du support intra-auriculaire (2) ;
une conduite de support intra-auriculaire (46) disposée entre ladite surface interne (7) et ladite surface externe (6) dudit support intra-auriculaire (2), ladite conduite de support intra-auriculaire (46) ayant une surface interne de conduite de support (47) conçue pour retenir de manière libérable un dispositif intra-auriculaire ; et
un matériau d'écouteur pouvant être moulé qui peut être moulé autour dudit support intra-auriculaire (2) et sur le contour du pavillon ou de l'arrondi de la conque de ladite oreille du porteur et éventuellement autour de ladite conduite de support intra-auriculaire (46) pour se conformer audit pavillon (9) ou audit arrondi de la conque de ladite oreille du porteur, ledit matériau d'écouteur (5) pouvant être moulé pouvant être durci dans une configuration fixe dudit écouteur (1).

3. Écouteur (1) selon la revendication 2, comprenant en outre :
un élément de saillie (3) s'étendant vers l'extérieur de ladite surface interne (7) dudit support intra-auriculaire (2) ; et
un corps souple (4) disposé autour dudit élément de saillie (3), ledit corps souple (4) pouvant être disposé à l'intérieur d'un conduit auditif externe d'une oreille en même temps que ledit support intra-auriculaire (2) est en prise avec un pavillon (9) de ladite oreille.

4. Écouteur (1) selon la revendication 2, dans lequel ladite conduite intra-auriculaire (46) s'étend à une distance vers l'extérieur de ladite surface externe (6) dudit support intra-auriculaire (2).

5. Écouteur (1) selon l'une quelconque des revendications 2, 3, 4, comprenant en outre un dispositif intra-auriculaire retenu de manière libérable par ladite conduite de support intra-auriculaire (46).

6. Écouteur (1) selon l'une quelconque des revendications 1 ou 2, dans lequel ledit matériau d'écouteur (5) pouvant être moulé comprend un matériau d'écouteur pouvant être moulé d'une seule pièce.

7. Écouteur (1) selon la revendication 6, dans lequel ledit matériau d'écouteur pouvant être moulé d'une seule pièce est choisi dans le groupe constitué par : l'acétate d'éthyle et de vinyle, la polycaprolactone, et la silicone à vulcanisation à température ambiante.

8. Écouteur (1) selon la revendication 7, dans lequel ledit matériau d'écouteur pouvant être moulé d'une seule pièce est chauffé pour obtenir un état pouvant être moulé afin de se conformer audit pavillon (9) de ladite oreille dans ladite configuration fixe dudit écouteur.

9. Écouteur (1) selon la revendication 8, dans lequel ledit matériau d'écouteur pouvant être moulé d'une seule pièce comprend une quantité de polymère de polycaprolactone ayant une masse moléculaire moyenne en nombre d'environ 37 000 grammes par mole à 80 000 grammes par mole.

10. Écouteur (1) selon l'une quelconque des revendications 1 ou 2, dans lequel ledit matériau d'écouteur (5) pouvant être moulé comprend un agent pouvant être moulé et un agent de durcissement, dans lequel l'association dudit agent pouvant être moulé et dudit agent de durcissement peut être durcie dans ladite configuration fixe dudit écouteur.

11. Écouteur (1) selon la revendication 10, dans lequel ledit agent pouvant être moulé et ledit agent de durcissement comprennent un polymère réticulable et un catalyseur.

12. Écouteur (1) selon la revendication 11, dans lequel ledit polymère réticulable comprend un polymère contenant au moins un groupement silane hydrolysable et dans lequel ledit catalyseur comprend un ou plusieurs parmi un sel métallique d'un acide carboxylique organique.

13. Écouteur (1) selon la revendication 11, dans lequel ledit polymère réticulable comprend un ou plusieurs parmi les polyoxyalkylènes, polyoléfines, poly(méth)acrylates, polyuréthanes, polyamides et polysiloxanes modifiés par silane et ledit sel métallique d'un acide carboxylique organique comprend un ou plusieurs parmi un sel de platine, d'étain ou de cuivre dudit acide carboxylique organique.

14. Écouteur (1) selon la revendication 11, dans lequel ledit polymère réticulable comprend du polydiméthylsiloxane et dans lequel ledit catalyseur comprend le (O)-1,3-divinyl-1,1,3,3-tétraméthyldisiloxane de platine.

15. Écouteur (1) selon l'une quelconque des revendications 1 ou 2, comprenant en outre un ou plusieurs éléments d'ouverture communiquant entre ladite surface externe (6) et ladite surface interne (7) dudit support intra-auriculaire (2).

16. Écouteur (1) selon l'une quelconque des revendications 1 ou 2, comprenant en outre des éléments adhésifs disposés sur ledit support intra-oculaire (2), lesdits éléments adhésifs étant conçus pour augmenter l'adhérence dudit matériau d'écouteur (5) pouvant être moulé audit support intra-auriculaire (2).
